# EUROPEAN PATENT SPECIFICATION

(11) **EP 3 448 352 B1**
(45) Date of publication and mention of the grant of the patent: **01.09.2021**
(21) Application number: 17790103.0
(22) Date of filing: 12.04.2017
(51) Int. Cl.: A61K 8/34, A61K 8/92, A61K 8/36, A61Q 5/00, A61Q 19/10

(54) **CLEANSING COMPOSITIONS AND METHODS**
REINIGUNGSZUSAMMENSETZUNGEN UND VERFAHREN
COMPOSITIONS ET PROCÉDÉS DE NETTOYAGE

(30) Priority: 27.04.2016 US 201615139955
(43) Date of publication of application: 06.03.2019
(73) Proprietor: ELC Management LLC, Melville, NY 11747 (US)
(72) Inventor: RANIERI-CHAMBERLAIN, Alaine, Holtsville, New York 11742 (US); CARRANO, Jacqueline, East Islip, New York 11730 (US); MESSIN, Steven, Plainview, New York 11803 (US); TOBIN, James, Hicksville, New York 11801 (US)
(74) Representative: Dehns
(86) International application number: PCT/US2017/027124
(87) International publication number: WO 2017/189231

(56) References cited:
- EP-B1- 1 754 518
- EP-B2- 1 331 914
- US-A1- 2007 027 050
- US-A1- 2007 196 403
- US-A1- 2015 118 328
- US-B1- 6 287 580

## Description

### Technical Field

The invention is in the field of cleansing compositions for keratin surfaces such as skin, nails or hair that may be in multiple phases.

### Background of the Invention

When cleansing skin or hair it is very desirable to use heat. When a cleansing composition is warmed the cleansing components often work more effectively. In addition, the feeling of warmth provides the consumer with a pleasant aesthetic that makes the cleansing process more enjoyable.

However, there are only a few ingredients that will provide a warming effect exactly when it is desired and not at other times. Zeolites are well known for this purpose. However, there are complications when formulating with zeolites. Products that contain zeolites require hermetic packaging because zeolites are extremely sensitive to air and immediately begin warming as soon as they are in contact with air. In addition, zeolites are not suitable formulating into topically applied compositions.

Thus there is a need for cleansing compositions that auto-warm and do not contain zeolites, but rather auto-warm due to the natural properties of the ingredients present.

It is an object of the invention to provide a multiple phase auto-warming composition for cleansing skin, nails, or hair; which does not contain zeolites.

It is another object of the invention to provide a multiple phase auto-warming composition that is free of preservatives.

It is another object of the invention to provide a multiple phase auto-warming composition that, when shaken to emulsify, remains emulsified from 1 to 60 minutes before separating into separate phases.

It is an object of the invention to provide a method for cleansing keratin surfaces with an auto-warming composition containing at least one fast blooming surfactant.

It is an object of the invention to provide a method for cleansing a keratin surface by applying an emulsified auto-warming composition to the dry keratin surface and adding water in an amount sufficient to cause the composition to auto-warm and lather. Because the cleansing composition of the invention has multiple phases it is able to dissolve and/or solvate many of the ingredients found in makeup as well as the particles and soil that may be deposited on the skin due to pollution or environmental conditions.

US2007/0196403A discloses an anhydrous cosmetic composition comprising: an inorganic heat generating agent which generates heat by mixing with water, from about 0.1 wt.% to about 10 wt.% of a polyoxyalkylene derivative, from about 0.2 wt.% to about 20 wt.% of a phase-changing material, from about 0.1 wt.% to about 10 wt.% of a cationic conditioning agent, and an inert carrier.

### Summary of the Invention

The invention is directed to a multiple phase auto-warming composition free of water, and zeolites as defined in claim 1 comprising a visually distinct alkoxylated glycerin phase and a visually distinct oil phase.

The invention is also directed to a method for cleansing skin or hair comprising contacting the skin or hair with a multiple phase auto-warming composition of the invention in the presence of sufficient extraneous water to cause the composition to auto-warm and activate the fast blooming surfactants.

The invention is also directed to a method for cleansing a keratin surface comprising:
(a) shaking a multiple phase auto-warming composition of the invention for a time sufficient to cause the multiple phases to combine into one phase;
(b) applying the composition of (a) to the dry keratin surface;
(c) adding water in an amount sufficient to cause the composition to auto-warm and the fast blooming surfactants to lather and emulsify with the added water.

The invention is also directed to a method for double cleansing a keratin surface comprising:
(a) shaking a multiple phase auto-warming composition of the invention for a time sufficient to cause the multiple phases to combine into one phase;
(b) applying the composition of (a) to the dry keratin surface;
(c) adding water in an amount sufficient to cause the composition to auto-warm and the fast blooming surfactants to lather and emulsify with the added water
(d) rinsing the composition of (c) from the keratin surface with water,
(e) applying a second aqueous based cleansing composition comprising water and at least one cleansing surfactant to the keratin surface and cleansing for at least 1 to 60 seconds; and
(f) rinsing the composition of ( e) with water.

### Detailed Description

### I. Definitions

The term "auto-warming" means that the composition increases in temperature at least 5° C. and up to about 15° C. upon exposure to water and without addition of any additional ingredients or chemicals (such as zeolites) that cause an increase in temperature. The temperature increase being measured from room temperature, 25° C.

The term "fast blooming" means a surfactant that is inactive in the stored composition but when exposed to water in amounts such as seen in washing skin or hair, rapidly (within 1 to 60 seconds), emulsifies with the water, lathers, and aids in removing dirt, soil and debris from a keratin surface.

The term "free of water" means that the composition contains less than 3% water, preferably less than 2% water, most preferably less than 0.6% water, or is anhydrous, meaning that the composition contains no intentionally added water.

The term "lather" means to form a froth or milky texture upon exposure to water.

The term "multiple phase" means at least two (or more) discrete and visible phases when the composition is at rest.

### II. The Alkoxylated Glycerin Phase

In the present invention, the alkoxylated glycerin is glycereth-26. The composition of the invention may comprise from 1 to 99%, preferably from 5 to 98%, more preferably from 10 to 95% alkoxylated glycerin. Most preferred is glycereth-26 having 26 ethoxy groups. Glycereth-26 is a clear to hazy viscous liquid at room temperature (25° C.) and may be purchased from a variety of sources including Lipo Chemicals Inc. under the trade name Liponic EG-1.

The alkoxylated glycerin phase may also contain other ingredients that are miscible with the alkoxylated glycerin and do not have an effect on its auto-warming property when combined with water. Examples of such ingredients include mono-, di-, or polyhydric alcohols or alkylene glycols such as glycerin, isopropanol, ethanol, ethylene glycol, propylene glycol, butylene glycol, sugar alcohols such as sorbitol, and the like. If present, these ingredients may range from about 0.01 to 4%.

### III. The Oil Phase

The composition may contain from about 1-65%, preferably from about 1-45%, more preferably about 5-35% oil. Suitable oils include those having a lower viscosity, ranging from 10-500 centipoise at 25° C. The oils should be pourable at room temperature. The oils are plant derived and are extracted from seeds, stems, flowers, leaves, or bark. Preferably the plant oils are in the form of glyceryl esters of fatty acids. Disclosed such oils include those from soybean, rapeseed, oat, shea butter, Camellia japonica, caprylic/capric triglycerides, safflower, caraway, pecan, quinoa, watermelon seed, coconut, coffee seed, hazel seed, cucumber, palm kernel, palm, cotton seed, canola, olive, peanut, sunflower seed, walnut, meadowfoam, linseed, mango seed, tea tree, rice germ, corn, poppy seed, avocado, apricot kernel, cherry seed, peach kernel, apple, black currant, sandalwood, tomato seed, vegetable oil, rice bran, carrot, coconut, kukui nut, olive, corn, palm, sunflower, and mixtures thereof.

### III. The Fast Blooming Surfactant

The composition contains at least one fast blooming surfactant present in the oil phase. The composition additionally comprises one or more fast blooming surfactants soluble in the oil phase. The surfactant may be present in amounts ranging from about 1 to 35%, more preferably from 2-30%, more preferably from 5-25%.
The fast blooming surfactant is selected from the group consisting of:
(a) a fatty acid ester of an alkylene glycol;
(b) a fatty acid ester of sorbitol or sorbitol anhydride;
(c) a fatty acid ester of hexatol anhydrides; and
(d) mixtures thereof.
Disclosed fast blooming surfactants include glyceryl fatty acid esters such as fatty acid esters of alkylene glycol ethers of glycerin, fatty acid esters of sorbitol or sorbitol anhydrides, alkylene glycols of fatty acid esters, fatty acid esters of hexatol anhydrides or mixtures thereof.

For example, PEG glyceryl fatty acid esters where the repeating ethylene glycol groups range from 1 to 100, preferably 5 to 50, more preferably from 10 to 30 PEG groups are disclosed. The fatty acid ester is preferably a C4-28 saturated or unsaturated ester of straight or branched chain alkyl group including but not limited to saturated fatty acids such as caprylic (C8), capric (C10), lauric (C12), myristic (C14), palmitic (C16), stearic (C18), arachidic (C20), behenic (C22), lignoceric (C24) and cerotic (C26). Suitable unsaturated fatty acids include myristoleic, palmitoleic, sapienic, oleic, elaidic, vaccenic, linoleic, arachidonic, eisopentaenoic, erucic, and docohexaenoic acids. The esters may be mono-, di-, or triesters of alkoxylated glyceryl fatty acids. Particularly preferred are PEG glyceryl fatty acid esters containing from 10-30, preferably 25 repeating ethylene glycol groups and where the fatty acid is oleic acid. Most preferred is PEG-25 glyceryl trioleate which is the tri-oleate ester of glycerin.

Also suitable as the fast blooming surfactants are fatty acid esters of sorbitol or sorbitol anhydrides. Examples include Polysorbates which are mixtures of oleate esters of sorbitol and sorbitol anhydrides which are ethoxylated with from about 1 to 100 ethoxy groups. Examples include Polysorbates 20, 21, 40, 60, 61, 65, 80, 81, 85, wherein the integers refer to the number of repeating ethoxy groups. Most preferred is Polysorbate 85.

Other disclosed fast blooming surfactants include alkylene glycol mono-, di, or tri-fatty acids. Preferred is where the alkylene glycol group is ethylene glycol. The fatty acids are the same as set forth above with respect to the alkylene glycol glyceryl fatty acid esters. Preferred is where the alkylene glycol fatty acid esters are PEG mono- or di-fatty acid esters. Most preferred are PEG fatty acid esters containing from 1 to 100 ethylene glycol groups and where the fatty acid ester is a diester. More preferred is where the fatty acid ester is a PEG fatty acid ester such as PEG diisostearate where the repeating ethoxy groups are from 1 to 20, more preferably 12. Most preferred is PEG-12 diisostearate.

Other suitable fast blooming surfactants include fatty acid esters of hexatol anhydrides, such as sorbitan where the esters may be mono-, di- or triesters. Suitable fatty acids are those mentioned above with respect to the alkoxylated glyceryl fatty acid esters. Most preferred is where the sorbitan esters are of hexatol anhydrides and oleic acid. Most preferred is Sorbitan trioleate.

### IV. Other Ingredients

The composition may also contain other ingredients so long as they do not interfere with the auto-warming property or negatively impact the activity of the fast blooming surfactant. In another embodiment of the invention the composition may be preservative free. That term means that the composition may be free of preservatives such as parabens.

It may be desireable to include one more colorants which may be in the form of organic or inorganic pigments. Suitable organic pigments include FD&C and D&C colorants and Lakes thereof. Inorganic pigments may include iron oxides. If present, colorants may range from 0.0001 to 5%. Most preferred is where the colorants present are D&C or FD&C colorants.

Fragrance oils may also be present, if desired. Generally fragrance oils will form part of the oil phase of the multi-phase composition. If present, suggested ranges are from about 0.1 to 5%, preferably from about 0.5 to 3%.

Antioxidants and preservatives may also be present. Suggested ranges are from 0.01 to 2% and may include tocopheryl derivatives such as tocopheryl acetate. Also suitable are benzophenone derivatives such as Benzophenone 4.

### V. The Composition

The composition is free of water. The term "free of water" means that the composition contains less than 2%, preferably less than 1%, most preferably less than 0.6% water. In one preferred embodiment the composition is anhydrous, which means that water is not intentionally added. A very small amount of water may be added in order to solvate water soluble ingredients that are desired to be added to the composition, for example, water soluble colorants. However, if too much water is present the composition auto-warms and the fast blooming surfactant will activate in the container before it can be used by the consumer.

In a preferred embodiment, the composition is also blendable, which means that when shaken to emulsify it will remain emulsified from 1 to 60 minutes, preferably from 5 to 45 minutes, more preferably from 8 to 35 minutes before separating into visually distinct multiple phases. In the most preferred embodiment the composition has two phases: the oil phase which resides on the top and the water phase which forms the bottom layer of the two phase composition.

The preferred composition of the invention is able to show a temperature increase of 5 to 20° C., preferably from 5 to 10° C. when contacted with sufficient water similar to what is seen when splashing the face with water during face washing. The temperature increase is measured from room temperature, 25° C. More specifically, the preferred composition of the invention will show a temperature increase ranging from 5 to 15° C. when 300 grams of the composition is combined with 100 grams of water with the temperature increase occurring in a period of time ranging from 10 to 60, preferably 30 to 60, more preferably from 45 to 60 seconds. The ratio of composition to water to provide such temperature increase may range from 2-4 parts composition to 2-4 parts water.

The composition may also be free of preservatives and zeolites which means that neither one of these ingredients are intentionally added. Given that water is required for growth of microorganisms, the little to no water present in the composition means that it is not conducive to growth of the microorganisms that require water to grow.

The composition is also free of zeolites, which means that zeolites are not intentionally added.

### VI. The Method

The invention is also directed to a method for cleansing a keratin surface comprising:
(a) shaking a multiple phase auto-warming composition of the invention for a time sufficient to cause the multiple phases to combine into one phase;
(b) applying the composition of (a) to the dry keratin surface;
(c) add water in an amount sufficient to cause the composition to auto-warm and the fast blooming surfactants to lather and emulsify with the added water to cleanse the keratin surface.

The composition is shaken to emulsify and blend the multiple phases which are in the case of a two phase composition, oil and water. When the composition has been emulsified it is applied to the dry keratin surface which may be skin, hair, or nails. If desired, prior to application of the composition to the dried keratin surface it may be dispensed into the palms of the hands and warmed prior to application to the keratin surface. Digital application may be used. The composition may be blended into the dry keratin surface by rubbing with the fingers. Then water is added in the usual manner that occurs with washing, such as splashing. When sufficient water contacts the composition on the keratin surface, the composition begins to auto-warm and the temperature increases from 5 to 15° C. on the keratin surface. In addition, the fast blooming surfactants react with the added water and activate to emulsify and form a "milk" which is a form of lather much like what is seen in cleansers referred to as cleansing milks. The composition is used to clean the keratin surface, then rinsed off with water. The increased temperature and cleansing surfactants improve the removal of soil, skin debris, makeup and the warm feel provides a pleasant consumer aesthetic.

The invention will be further described in connection with the following examples which are set forth for the purposes of illustration only.

### EXAMPLE 1

Two phase cleanser formulas were made as follows:

| Ingredient | Wt% | |
|---|---|---|
| Glycereth-26 (aqueous phase) | QS | QS |
| Soybean oil (oil phase) | 27.9 | 31.5 |
| PEG-25 glyceryl trioleate (oil phase) | 10.00 | 12.00 |
| Polysorbate 85 (oil phase) | 8.00 | 9.60 |
| PEG-12 diisostearate (oil phase) | 2.00 | 2.40 |
| Oryza sativa (rice) bran oil (oil phase) | 1.00 | 1.20 |
| Sorbitan trioleate (oil phase) | 1.0 | 1.20 |
| Water (aqueous phase) | 0.5 | 0.40 |
| Benzophenone-4 (aqueous phase) | 0.15 | 0.12 |
| Tocopheryl acetate (oil phase) | 0.10 | 0.12 |
| Butylene glycol (aqueous phase) | 0.06 | 0.03 |
| Colorants (aqueous phase) | 0.060 | 0.001 |
| Fragrance (oil phase) | -- | 2.00 |

The soybean oil, PEG-25 glyceryl dioleate, *Oryza sativa* oil, sorbitan trioleate, tocopheryl acetate, Polysorbate 85, and PEG-12 diisostearate were combined and mixed well. The water, Benzophenone-4 and Glycereth-26 were then added and mixed well. The colorants and fragrance were added last. The compositions were placed into glass jars. They separated into two phases. The aqueous phase formed the bottom phase and the oil phase the top phase.

### EXAMPLE 2

The auto-warming of the compositions was tested. Two separate formulas representing the Oil Phase and Alkoxylated Glycerin Phase were prepared as follows:
A formula referred to as "Oil Phase" was prepared as follows:

| Ingredient | Wt% |
|---|---|
| Soybean Oil | QS100 |
| PEG-24 glyceryl trioleate | 20.00 |
| Polysorbate 85 | 16.00 |
| PEG-12 diisostearate | 4.00 |
| Fragrance | 3.34 |
| Oryza sativa (Rice) bran oil | 2.00 |
| Tocopheryl acetate | 0.20 |

A second formula referred to as "Alkoxylated Glycerin Phase" was prepared as follows:

| Ingredient | Wt% |
|---|---|
| Glycereth-26 | QS100 |
| Water | 0.95 |
| Benzophenone-4 | 0.20 |
| Butylene glycol | 0.07 |

Using a Heidolph prop mixer at speed 2, a total of 300 grams of the Oil Phase ("O") and/or Alkoxylated Glycerin Phases ("AG") were combined in varying ratios as set forth below, and the change in temperature over time was measured. The temperature was recorded after it had stabilized and the amount of time in seconds that it took to stabilize was also measured.

| | 100% O | 100% AG | 99% AG 1%O | 35% AG 65% O | 15% AG 85% O | 10% AG 90% O | 5% AG 95% O | 1% AG 99% O |
|---|---|---|---|---|---|---|---|---|
| Initial Temperature °C. | 21.9 | 21.4 | 21.4 | 22.0 | 22.5 | 22.6 | 22.0 | 22.1 |
| Final temperature (after addition of 100 grams DIH₂O) | 26.2 | 35.6 | 34.7 | 32.7 | 30.2 | 29.4 | 27.0 | 27.0 |
| Change in temperature °C. | +4.3 | +14.2 | +13.3 | +10.7 | +7.7 | +6.8 | +5.0 | +6.0 |
| Time to reach final temperature in seconds | 47 | 47 | 45 | 50 | 48 | 58 | 48 | 41 |

The results demonstrate that adding 100 grams of water to the Oil Phase provides a very minimal change in temperature (4.3° C.) which is almost imperceptible. However, adding 100 grams of water to the AG phase provides a significant temperature increase over time. Varying the ratios of O an AG phases demonstrates that the amount of the AG phase that is present directly correlates with the temperature increase as the amount of the AG phase increases the temperature increase. A composition that contains sufficient oil phase to promote cleansing and sufficient AG phase to promote cleansing and warmth is most desirable.

## Claims

1. A multiple phase auto-warming composition free of water and zeolites comprising at least one visually distinct alkoxylated glycerin phase and at least one visually distinct oil phase containing at least one fast blooming surfactant,
wherein the alkoxylated glycerin is glycereth-26,
wherein the oil phase comprises a plant oil obtained from seeds, stems, flowers, leaves or bark,
wherein the composition additionally comprises one or more fast blooming surfactants soluble in the oil phase,
and wherein the fast blooming surfactant is selected from the group consisting of:
(a) a fatty acid ester of an alkylene glycol;
(b) a fatty acid ester of sorbitol or sorbitol anhydride;
(c) a fatty acid ester of hexatol anhydrides; and
(d) mixtures thereof.

2. The composition of claim 1 wherein the alkoxylated glycerin phase is present from 1-99% by weight of the total composition.

3. The composition of claim 2 wherein the oil phase is present from 1 to 99% by weight of the total composition.

4. The composition of claim 1 which auto-warms at least 5 to 20° C. upon contact with water, preferably which auto-warms in 5 to 60 seconds after contact with water.

5. The composition of claim 1 wherein the oil phase comprises a plant oil obtained from seeds, stems, flowers, or leaves.

6. The composition of claim 1 or claim 5 wherein the plant oil is a glyceryl ester of a fatty acid.

7. The composition of claim 1 wherein the alkylene glycol is ethylene glycol, and preferably wherein the fatty acid ester is a C6-22 saturated or unsaturated fatty acid ester.

8. The composition of claim 7 wherein the fatty acid ester is oleic acid, stearic acid, or mixtures thereof.

9. The composition of claim 8 wherein the ethylene glycol has from 10 to 100 ethylene glycol groups and the fatty acid is C6-22 fatty acid ester.

10. A method for cleansing a keratin surface comprising:
(a) shaking a multiple phase auto-warming composition as defined in claim 1 for a time sufficient to cause the multiple phases to combine into one phase;
(b) applying the composition of (a) to the dry keratin surface;
(c) adding water in an amount sufficient to cause the composition to auto-warm and the fast blooming surfactants to lather and emulsify with the added water to cleanse the keratin surface.

11. The method of claim 10 wherein the keratin surface is skin, preferably wherein after step (a) the composition is warmed in the palms of the hands prior to application to the dry keratin surface.

## Patentansprüche

1. Mehrphasige selbsterwärmende Zusammensetzung frei von Wasser und Zeolithen, die mindestens eine visuell eindeutige alkoxylierte Glycerinphase und mindestens eine visuell eindeutige Ölphase, enthaltend mindestens ein schnell ausblühendes Tensid, umfasst,
wobei das alkoxylierte Glycerin Glycereth-26 ist,
wobei die Ölphase ein aus Samen, Stängeln, Blüten, Blättern oder Rinde gewonnenes pflanzliches Öl umfasst,
wobei die Zusammensetzung zusätzlich ein oder mehrere schnell ausblühende Tenside, die in der Ölphase löslich sind, umfasst,
und wobei das schnell ausblühende Tensid ausgewählt ist aus der Gruppe, bestehend aus:
(a) einem Fettsäureester eines Alkylenglycols;
(b) einem Fettsäureester von Sorbitol oder Sorbitolanhydrid;
(c) einem Fettsäureester von Hexatolanhydriden; und
(d) Mischungen davon.

2. Zusammensetzung nach Anspruch 1, wobei die alkoxylierte Glycerinphase in einer Menge von 1-99 Gew.-% der Gesamtzusammensetzung vorhanden ist.

3. Zusammensetzung nach Anspruch 2, wobei die Ölphase in einer Menge von 1 bis 99 Gew.-% der Gesamtzusammensetzung vorhanden ist.

4. Zusammensetzung nach Anspruch 1, die sich bei Kontakt mit Wasser um mindestens 5 bis 20 °C selbst erwärmt, sich vorzugsweise in 5 bis 60 Sekunden nach Kontakt mit Wasser selbst erwärmt.

5. Zusammensetzung nach Anspruch 1, wobei die Ölphase ein aus Samen, Stängeln, Blüten oder Blättern gewonnenes pflanzliches Öl umfasst.

6. Zusammensetzung nach Anspruch 1 oder Anspruch 5, wobei das pflanzliche Öl ein Glycerylester einer Fettsäure ist.

7. Zusammensetzung nach Anspruch 1, wobei das Alkylenglycol Ethylenglycol ist und wobei der Fettsäureester vorzugsweise ein C6-22-gesättigter oder ungesättigter Fettsäureester ist.

8. Zusammensetzung nach Anspruch 7, wobei der Fettsäureester Ölsäure, Stearinsäure oder Mischungen davon ist.

9. Zusammensetzung nach Anspruch 8, wobei das Ethylenglycol 10 bis 100 Ethylenglycolgruppen aufweist und die Fettsäure C6-22-Fettsäureester ist.

10. Verfahren zum Reinigen einer Keratinoberfläche, umfassend:
(a) Schütteln einer mehrphasigen selbsterwärmenden Zusammensetzung nach Anspruch 1 für eine Zeit, die ausreicht, um zu bewirken, dass sich die mehreren Phasen zu einer Phase kombinieren;
(b) Aufbringen der Zusammensetzung von (a) auf die trockene Keratinoberfläche;
(c) Zugeben von Wasser in einer ausreichenden Menge, um zu bewirken, dass sich die Zusammensetzung selbst erwärmt und die schnell ausblühenden Tenside schäumen und mit dem zugegebenen Wasser emulgieren, um die Keratinoberfläche zu reinigen.

11. Verfahren nach Anspruch 10, wobei die Keratinoberfläche Haut ist, wobei nach Schritt (a) die Zusammensetzung vor dem Aufbringen auf die trockene Keratinoberfläche vorzugsweise in den Handflächen erwärmt wird.

## Revendications

1. Composition autochauffante à phases multiples exempte d'eau et de zéolites comprenant au moins une phase de glycérine alcoxylée visuellement distincte et au moins une phase huileuse visuellement distincte contenant au moins un tensioactif à développement rapide,
dans laquelle la glycérine alcoxylée est glycereth-26,
dans laquelle la phase huileuse comprend une huile végétale obtenue à partir de graines, de tiges, de fleurs, de feuilles ou d'écorce,
dans laquelle la composition comprend en plus un ou plusieurs tensioactifs à développement rapide solubles dans la phase huileuse,
et dans laquelle le tensioactif à développement rapide est choisi dans le groupe consistant en
(a) un ester d'acide gras d'un alkylène glycol ;
(b) un ester d'acide gras de sorbitol ou un anhydride de sorbitol ;
(c) un ester d'acide gras d'anhydrides d'hexatol ; et
(d) des mélanges de ceux-ci.

2. Composition selon la revendication 1 dans laquelle la phase de glycérine alcoxylée est présente de 1-99 % en poids de la composition totale.

3. Composition selon la revendication 2 dans laquelle la phase huileuse est présente de 1 à 99 % en poids de la composition totale.

4. Composition selon la revendication 1 qui s'autochauffe au moins 5 à 20 °C lors du contact avec l'eau, de préférence qui s'autochauffe en 5 à 60 secondes après le contact avec l'eau.

5. Composition selon la revendication 1 dans laquelle la phase huileuse comprend une huile végétale obtenue à partir de graines, de tiges, de fleurs, ou de feuilles.

6. Composition selon la revendication 1 ou la revendication 5 dans laquelle l'huile de plante est un ester glycéryle d'un acide gras.

7. Composition selon la revendication 1 dans laquelle l'alkylène glycol est un éthylène glycol, et de préférence dans laquelle l'ester d'acide gras est un ester d'acide gras saturé ou insaturé en C6-22.

8. Composition selon la revendication 7 dans laquelle l'ester d'acide gras est un acide oléique, un acide stéarique ou des mélanges de ceux-ci.

9. Composition selon la revendication 8 dans laquelle l'éthylène glycol présente de 10 à 100 groupes d'éthylène glycol et l'acide gras est un ester d'acide gras en C6-22.

10. Procédé de nettoyage d'une surface de kératine comprenant :
(a) le mélange d'une composition autochauffante à phases multiples telle que définie dans la revendication 1 pendant un temps suffisant pour amener les phases multiples à se combiner en une phase ;
(b) l'application de la composition de (a) sur la surface de kératine sèche ;
(c) l'ajout d'eau dans une quantité suffisante pour amener la composition à s'autochauffer et les tensioactifs à développement rapide à mousser et s'émulsifier avec l'eau ajoutée pour nettoyer la surface de kératine.

11. Procédé selon la revendication 10 dans lequel la surface de kératine est de la peau, de préférence dans lequel après l'étape (a), la composition est chauffée dans les paumes des mains avant application sur la surface de kératine sèche.
